# EUROPÄISCHE PATENTSCHRIFT

(11) **EP 1 401 804 B1**
(45) Veröffentlichungstag und Bekanntmachung des Hinweises auf die Patenterteilung: **14.03.2007**
(21) Anmeldenummer: 02726038.9
(22) Anmeldetag: 17.05.2002
(51) Int. Cl.: C07C 309/00, C07C 305/00, C12N 9/99, C12Q 1/30, G01N 7/18

(54) **KATALASE INAKTIVIERENDE VERBINDUNGEN UND DEREN VERWENDUNG**
CATALASE INACTIVATING COMPOUNDS AND THEIR USE
COMPOSES INACTIVANT LA CATALASE ET LEUR UTILISATION

(30) Priorität: 02.07.2001 CH 120501
(43) Veröffentlichungstag der Anmeldung: 31.03.2004
(73) Patentinhaber: Podzus, Bernd Fritz, 8447 Dachsen (CH)
(72) Erfinder: Podzus, Bernd Fritz, 8447 Dachsen (CH)
(74) Vertreter: Köver, François
(86) Internationale Anmeldenummer: PCT/CH2002/000270
(87) Internationale Veröffentlichungsnummer: WO 2003/004462

(56) Entgegenhaltungen:
- EP-A- 0 184 260
- WO-A-93/15218
- US-A- 5 610 025

## Beschreibung

### Technisches Gebiet

Die Erfindung betrifft Sulfonylester der allgemeinen Formel R₁-COO-SO₂-Z-R₂, worin Z, R₁ und R₂ die im Anspruch 1 definierten Bedeutungen haben, eine Verwendung dieser Verbindungen zur Modifikation der Kinetik der enzymatischen Wirkung von Katalase, ein Verfahren zur Messung einer Konzentration an lebenden und/oder aktiven Mikroorganismen wie Bakterien und/oder Pilze, insbesondere Schimmelpilze und/oder Hefen, in einer flüssigen Probe anhand der Entwicklung von Sauerstoff aus Wasserstoffperoxid, und eine Vorrichtung zur Ausführung dieses Verfahrens.

### Stand der Technik

Die Bestimmung der Keimbelastung von Lebensmitteln, Kühlschmierstoffen und dergleichen, oder auch die Überprüfung der Wirksamkeit eines Biozides oder Desinfektionsmittels, erfolgt derzeit überwiegend durch mikrobiologische Methoden, die sich eines Wachstums der zu bestimmenden Mikroorganismen bedienen und deshalb langsam sind und sich nicht zur laufenden Kontrolle der Keimbelastung vor Ort eignen.

Die wichtigsten Verursacher der Keimbelastung sind bei Lebensmitteln hauptsächlich aerobe Mikroorganismen (wie Bakterien und/oder Pilze, insbesondere Schimmelpilze und/oder Hefen) und bei wässrigen Kühlschmierstoff-Emulsionen auch fakultativ anaerobe Mikroorganismen. In allen Mikroorganismen, welche zur Energieerzeugung ein auf Cytochrom-Elektronentransport beruhendes Atmungssystem haben (und dies sind fast alle Mikroorganismen, die in Lebensmitteln und Kühlschmierstoff-Emulsionen vorkommen) ist ein bestimmtes, Katalase genanntes Enzym zu finden, das chemisch ein tetrameres Eisen-Protein (Mᵣ = 245000) mit 4 Häm-Mol in Form von Ferriprotoporphyrin IX mit Eisen(III) ist. Katalase spaltet ausserordentlich rasch Wasserstoffperoxid zu Wasser und Sauerstoff, wobei die Sauerstoffentwicklung direkt proportional zur Enzymkonzentration verläuft. Darauf beruht ein bekanntes Verfahren zur Bestimmung der Keimbelastung, das schneller ist als vom Mikroorganismenwachstum abhängige Verfahren - vgl. dazu H.K. Frank & U. Hertkorn-Obst, Chem. Mikrobiol. Technol. Lebensm. *6,* 143-149 (1980) und/oder R.G. Kroll, E.R. Frears & A. Bayliss, J. Appl. Bacteriol. *66,* 209-217 (1989). Die Messung der Sauerstoffentwicklung kann auf verschiedene bekannte Weisen erfolgen, unter anderem manometrisch - vgl. dazu G.J. Wang & D.Y.C. Fung, J. of Food Safety *8* , 46-47 (1986), sowie EP-476850. Es ist bekanntlich vorteilhaft, die zu bestimmenden Mikroorganismen in eine wässrige Probe zu bringen und Wasserstoffperoxid in solcher Menge zuzugeben, dass sich eine Konzentration von etwa 1 Vol.-% ergibt, wobei für die meisten zu bestimmenden Mikroorganismen der für die Messung optimale pH-Wert im neutralen Bereich bei 7 liegt.

Im übrigen sind, zur Bestimmung der Keimbelastung eines untersuchten Guts bzw. einer Probe davon, nur die lebenden Mikroorganismen zu erfassen, d.h. es ist nur die enzymatische Wirkung der aktiven intrazellulären Katalase dieser lebenden Mikroorganismen von Bedeutung und zu messen. Messfehler ergeben sich jedoch aus der enzymatischen Wirkung von in der Probe befindlicher aktiver endogener Katalase aus verschiedensten Quellen. So berichten sowohl H.K. Frank & U. Hertkom-Obst wie auch R.G. Kroll, E.R. Frears & A. Bayliss über diverse Schwierigkeiten. Es ist ein besonderer Aufwand nötig, um zwischen lebenden und toten Hefezellen (Bierhefe) zu unterscheiden. Lebensmittel enthalten nicht-mikrobielle endogene Katalase, die durch Pflanzenteile (Gemüsesalate, Zwiebel), Saft (Apfelsaft, Zitronensaft) und tierisches Gewebe (Erythrozyten in Fleisch und Milch) in die Proben eingebracht wird und durch ihre enzymatische Wirkung die Messung deutlich verfälscht, es sei denn, die Wirkung dieser nicht-mikrobiellen endogenen Katalase sei vorgängig beispielsweise durch Hitzebehandlung (Pasteurisierung, Konservierung) beseitigt worden. Auch Trinkwasser und Freiwasser enthalten störende Pflanzenreste und Schwebstoffe der verschiedensten Art, welche die Sauerstoffentwicklung bereits beim Einbringen des Wasserstoffperoxids in die Probe katalysieren, es sei denn, diese Störsubstanzen werden durch Filtration beseitigt.

Die vorstehend erwähnten Schwierigkeiten machen die derzeit zur Verfügung stehenden relativ schnellen Verfahren zur Bestimmung der Keimbelastung von Lebensmitteln, Kühlschmierstoffen und dergleichen wie auch zur Überprüfung der Wirksamkeit eines Biozides oder Desinfektionsmittels unzuverlässig.

Aus EP-184260 ist ein Verfahren bekannt, mit dem der gesamte Gehalt an Katalase, nicht aber die Konzentration an lebenden und/oder aktiven Mikroorganismen nachgewiesen wird. Bei diesem Verfahren wird sowohl die bakterielle wie auch die nichtbakterielle d.h. endogene Katalase erfasst, was zu wesentlichen Differenzen führt. Aufgrund des gestörten Zusammenhangs zwischen der Anzahl Kolonien bildender Einheiten (KbE) und den Katalase-Werten unter Biomasse-Einfluss ist somit nicht in jedem Fall eine Beurteilung der mikrobiellen Belastung bzw. die Messung der Konzentration an lebenden und/oder aktiven Mikroorganismen möglich.

Aus US-5610025 und/oder WO-93/15218 ist ein Verfahren bekannt, mit dem Wasserstoffperoxid zersetzende Enzyme in Gegenwart von Katalase nachgewiesen werden. Vor der Zugabe des Wasserstoffperoxids wird die Katalase durch Zugabe eines Hydroxylaminsalzes gehemmt. Zu einer Messung der Konzentration an lebenden und/oder aktiven Mikroorganismen führt diese Lehre nicht.

Aufgabe der Erfindung ist es demnach, ein Verfahren zur Messung einer Konzentration an lebenden und/oder aktiven Mikroorganismen zur Verfügung zu stellen, dessen Messwert-Ergebnisse zuverlässig sind und insbesondere nicht von endogener Katalase verfälscht werden.

Aufgabe der Erfindung ist es demnach auch, chemische Verbindungen aufzuzeigen und zur Verfügung zu stellen, welche eine Modifikation der Kinetik der enzymatischen Wirkung von Katalase bewirken, insbesondere eine vorzugsweise irreversible Inaktivierung der enzymatischen Wirkung von Katalase, insbesondere von aktiver endogener Katalase, insbesondere ohne merkliche Inaktivierung der enzymatischen Wirkung von aktiver intrazellulärer Katalase.

Aufgabe der Erfindung ist es ebenfalls, ein Verfahren der vorgenannten Art, dessen Messwert-Ergebnisse schnell erhältlich und interpretierbar sind, sowie eine sich dazu eignende Vorrichtung zur Verfügung zu stellen.

### Darstellung der Erfindung

Zur Lösung dieser und weiterer Aufgaben, die aus der nachstehenden Beschreibung der Erfindung erkennbar sind, werden erfindungsgemässe neue chemische Verbindungen angegeben und deren erfindungsgemässe Verwendungen in den entsprechenden Stoffansprüchen und Verwendungsansprüchen definiert.

Ebenfalls sind ein erfindungsgemässes Verfahren und bevorzugte Weiterbildungen davon in den entsprechenden Verfahrensansprüchen sowie eine bevorzugte erfindungsgemässe Vorrichtung zur Ausführung des erfindungsgemässen Verfahrens und bevorzugte Weiterbildungen davon in den entsprechenden Vorrichtungsansprüchen definiert.

Dank der Einfachheit des erfindungsgemässen Verfahrens insbesondere im Zusammenhang mit der erfindungsgemässen Vorrichtung zu dessen Ausführung ermöglicht die Erfindung, das Verfahren leicht, schnell und kostengünstig auszuführen, wobei eine direkte Anzeige einer Konzentration an lebenden und/oder aktiven Mikroorganismen ermöglicht wird.

Da beim erfindungsgemässen Verfahren sowohl die Reaktionszeit als auch die Messzeit gering sind, besteht beispielsweise die Möglichkeit, ein Lebensmittel direkt vor der Vermarktung oder kurz vor dem Verzehr einer Kontrolle zu unterziehen, um Chargen mit zu hoher Keimbelastung noch rechtzeitig zurückhalten zu können.

Insbesondere sind die Messwert-Ergebnisse des erfindungsgemässen Verfahrens in kürzerer Zeit als etwa einer halben Stunde und deshalb aktuell (praktisch in Echtzeit) erhältlich, was deren Verwendung vor Ort bei der Steuerung und/oder Kontrolle eines Verfahrens ermöglicht, wobei die aktive intrazelluläre Katalase diejenige von lebenden Mikroorganismen wie Bakterien und/oder Pilze, insbesondere Schimmelpilze und/oder Hefen, ist.

Zudem bewirken die von der Erfindung aufgezeigten und zur Verfügung gestellten chemischen Verbindungen eine Modifikation der Kinetik der enzymatischen Wirkung von Katalase, welche deren Verwendung zur Abtötung von Mikroorganismen ermöglicht.

### Kurze Beschreibung der Zeichnungen

Ausführungsbeispiele der Erfindung werden nachfolgend anhand der Zeichnungen näher beschrieben. Dabei zeigen:
- Figur 1 bis 8: aufeinanderfolgende Schritte eines erfindungsgemässen Verfahrens anhand schematischer Darstellungen von dazu verwendeten Gegenständen und Flüssigkeiten;
- Figur 9: ein schematisch dargestelltes Druckmessgerät einer Vorrichtung zur Ausführung des erfindungsgemässen Verfahrens mit einem Drucksondenteil und einem Druckauswerteteil in jeweils schematischer Darstellung;
- Figur 10: ein schematisch dargestelltes Entlüftungswerkzeug einer Vorrichtung zur Ausführung des erfindungsgemässen Verfahrens; und
- Figur 11: einen schematisch dargestellten Stopfen zur Verwendung mit einer Vorrichtung zur Ausführung des erfindungsgemässen Verfahrens.

### Wege zur Ausführung der Erfindung

Es wurde im Rahmen der Erfindung gefunden, dass gewisse chemische Verbindungen fähig sind, die Kinetik der enzymatischen Wirkung von Katalase zu modifizieren. Diese Modifikation kann bei aktiver endogener Katalase oder aktiver intrazellulärer Katalase verschieden sein. In Abhängigkeit der verwendeten chemischen Verbindung und des Typus der davon modifizierten Katalase (endogen oder intrazellulär, in letzterem Fall in Zellen verschiedener Mikroorganismen vorliegend) kann sich diese Modifikation als irreversible Inaktivierung der gesamten Katalase auswirken, und/oder es kann eine irreversible Inaktivierung von aktiver endogener Katalase ohne merkliche Inaktivierung der enzymatischen Wirkung von aktiver intrazellulärer Katalase erreicht werden. Daraus ergibt sich eine Vielfalt von Anwendungen, unter denen die Messung einer Konzentration an lebenden und/oder aktiven Mikroorganismen und/oder die Abtötung von Mikroorganismen als von besonderem Interesse sind.

Die in diesem Zusammenhang in Frage kommenden Mikroorganismen können Bakterien und/oder Pilze, insbesondere Schimmelpilze und/oder Hefen sein.

Die in diesem Zusammenhang in Frage kommenden chemischen Verbindungen sind Sulfonylester der allgemeinen Formel

R₁-COO-SO₂-Z-R₂

worin
- Z: für Phenylen steht oder abwesend ist,
- R1: über ein primäres oder sekundäres Kohlenstoffatom mit dem Kohlenstoffatom des Säureradikals -COO- verbunden ist und für geradkettiges oder verzweigtes Alkyl mit 2 bis 21000 Kohlenstoffatomen, geradkettiges oder verzweigtes Alkenyl mit 2 bis 21000 Kohlenstoffatomen, oder geradkettiges oder verzweigtes Alkinyl mit 2 bis 21000 Kohlenstoffatomen steht, und
- R₂: über ein sekundäres oder tertiäres Kohlenstoffatom mit dem Radikal Phenylen in Position ortho, meta oder para zum Radikal SO₂ wenn Z vorhanden ist, oder mit dem Radikal SO₂, wenn Z abwesend ist, verbunden ist und für geradkettiges oder verzweigtes Alkyl mit 3 bis 100 Kohlenstoffatomen, geradkettiges oder verzweigtes Alkenyl mit 3 bis 100 Kohlenstoffatomen, geradkettiges oder verzweigtes Alkinyl mit 3 bis 100 Kohlenstoffatomen, geradkettiges oder verzweigtes Alkoxy mit 3 bis 100 Kohlenstoffatomen, geradkettiges oder verzweigtes Hydroxyalkyl mit 3 bis 100 Kohlenstoffatomen,
wobei das Radikal Hydroxy über ein primäres oder sekundäres Kohlenstoffatom mit dem Radikal Alkyl verbunden ist, oder
Polyoxaalkyl mit 3 bis 100 Kohlenstoffatomen steht.

Unter diesen Verbindungen sind unter anderem bevorzugt: 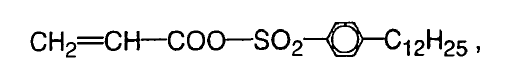 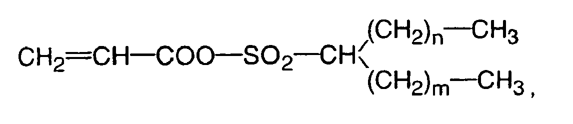 worin n und m natürliche Zahlen sind und 3 < (n+m) < 60 ,

CH₂=CH-COO-SO₂-CH₂-CH=CH-C₁₃H₂₇,

CH₂=CH-COO-SO₂-O-C₁₂H₂₅,

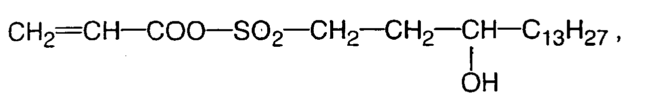

und

CH₂=CH-COO-SO₂-O-CH₂-CH₂-O-CH₂-CH₂-O-C₁₂H₂₅.

### Herstellung und Untersuchung der Verbindung

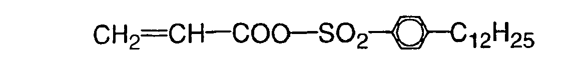

In einen 1000-ml-Erlenmeyer-Kolben werden 460 ml destilliertes Wasser gegeben und dann 94 g Dodecylbenzolsulfonsäure Natriumsalz zugegeben. Bei etwa 50°C wird mit einem Magnetrührer bis zur vollständigen Auflösung gerührt, dann wird die Lösung etwa 15 Minuten zum Abkühlen stehengelassen. Daraufhin werden 2,4 ml wasserfreie Acrylsäure langsam unter Rühren zugegeben, und die Lösung wird weiter gerührt, bis sie die Raumtemperatur erreicht. Nach Stehenlassen dieser Lösung während mindestens 24 Stunden im Dunkeln bei Raumtemperatur werden in einen 250-ml-Erlenmeyer-Kolben 79,5 ml destilliertes Wasser gegeben und dann 0,5 ml einer 0,066 M Phosphatpufferlösung (pH-Wert 7,00) unter Rühren zugegeben. Anschliessend wird 1 g des am Vortag erhaltenen Lösungsgemisches unter Rühren zugegeben. Die so erhaltene Lösung der Titelverbindung hat einen pH-Wert von etwa 4,6.

Als Reagenzien werden bereitgestellt:
Katalase aus Rinderleber (als aktive endogene Katalase)
Katalase aus Aspergillus niger (als aktive intrazelluläre Katalase)
Katalase aus Mikroorganismen^{(*)} (als aktive intrazelluläre Katalase) ^{(*)} diverse Bakterien und/oder Pilze
Entschäumer (RD^{®} von Dow Coming)
Wasserstoffperoxid (Perhydrol^{®} 30% von Merck)

Jede Probe enthält pro 1 ml destilliertes Wasser jeweils 35 Einheiten Katalase aus Rinderleber oder aus Aspergillus niger oder aus Mikroorganismen. Die 1-ml-Probe wird in ein 15-ml-Röhrchen (Vacutainer^{®} 16 x 125 mm von Becton Dickinson) gegeben, jeweils mit 7 ml der vorgenannten Lösung der Titelverbindung versetzt und damit vermischt (Mischgerät Vortex^{®} Genie 2 von Merck). Dann wird 1 Tropfen des genannten Entschäumers zugegeben und erneut vermischt. Danach werden 0,23 g des genannten Wasserstoffperoxids zugegeben, und das Röhrchen wird sofort mit einem Stopfen aus chemikalienbeständigem elastischem Kunststoff (Viton^{®} von Du Pont de Nemours) gasdicht verschlossen, mit Hilfe einer den Stopfen durchstechenden Hohlnadel (Injektionsnadel Ø 1 mm) kurz (wenige Sekunden) entlüftet und gleich danach durch Zurückziehen der Hohlnadel aus dem Stopfen wieder gasdicht verschlossen. Das gasdicht verschlossene Röhrchen wird bei Raumtemperatur während 15 Minuten stehengelassen. Danach wird das horizontal gekippte Röhrchen 10mal hin und her geschüttelt, um sicherzustellen, dass die Zellmembranen aufplatzen. Dann wird der Druck im Röhrchen gemessen (vgl. weiter unten die Beschreibung des bevorzugten Druckmessers).

Es wird festgestellt, dass sich der Druck im Röhrchen nicht erhöht. Daraus folgt, dass die Titelverbindung sowohl aktive endogene Katalase als auch aktive intrazelluläre Katalase inaktiviert. Daraus folgt auch, dass die Titelverbindung zur Abtötung von Mikroorganismen verwendbar ist, da diese Mikroorganismen, deren intrazelluläre Katalase inaktiviert ist, wegen des Fehlens der Wirkung dieser Katalase absterben.

### Herstellung und Untersuchung der Verbindung

CH₂=CH-COO-SO₂-O-C₁₂H₂₅

In einen 500-ml-Erlenmeyer-Kolben werden 200 ml destilliertes Wasser gegeben und dann 25 g Natriumlaurylsulfat zugegeben. Bei etwa 70°C wird mit einem Magnetrührer bis zur vollständigen Auflösung gerührt. Unter Beibehaltung der Temperatur werden daraufhin 1,07 g Polyacrylsäure (Mᵣ = 500000 bis 1000000) langsam unter Rühren zugegeben, und die Lösung wird während 45 Minuten weiter gerührt. Anschliessend werden 5 ml einer 0,25 M Lösung von Dinatriumhydrogenphosphat-Dihydrat in destilliertem Wasser zugegeben, und dann werden alle 15 Minuten jeweils 2 ml der Lösung nachdosiert, bis insgesamt 12 ml der Lösung verbraucht sind. Das erhaltene Lösungsgemisch wird zunächst unter Beibehaltung der Temperatur während 60 Minuten weiter gerührt und dann auf Raumtemperatur gekühlt. Das so erhaltene Lösungsgemisch hat einen pH-Wert von etwa 5,9. Nun werden in einen 250-ml-Erlenmeyer-Kolben 42 ml destilliertes Wasser gegeben und dann 5 ml einer 0,066 M Phosphatpufferlösung (pH-Wert 7,00) unter Rühren zugegeben. Anschliessend werden 3 g des vorhin erhaltenen Lösungsgemisches unter Rühren zugegeben. Die so erhaltene Lösung der Titelverbindung hat einen pH-Wert von etwa 6,4.

Es werden die gleichen Reagenzien bereitgestellt, die bereits im vorangehenden beschrieben wurden, und es wird auf gleiche Weise wie im vorangehenden die Katalase der Wirkung der Titelverbindung ausgesetzt und die sich danach ergebende Aktivität der Katalase über die Sauerstoffentwicklung (Druckerhöhung) untersucht.

Wenn in der Probe nur aktive endogene Katalase vorliegt, wird festgestellt, dass sich der Druck im Röhrchen nicht erhöht. Wenn jedoch in der Probe auch aktive intrazelluläre Katalase aus Mikroorganismen wie Bakterien und/oder Pilze, insbesondere Schimmelpilze und/oder Hefen, vorliegt, wird festgestellt, dass sich der Druck im Röhrchen erhöht. Daraus folgt, dass die Titelverbindung aktive endogene Katalase inaktiviert, jedoch aktive intrazelluläre Katalase nicht merklich inaktiviert.

### Verwendungen der erfindungsgemässen Verbindungen

Die vorangehenden Beispiele von Herstellung und Untersuchung von erfindungsgemässen chemischen Verbindungen zeigen auf, dass letztere eine Modifikation der Kinetik der enzymatischen Wirkung von Katalase bewirken. Es hat sich gezeigt, dass sich je nach der verwendeten erfindungsgemässen Verbindung und je nach den damit behandelten Mikroorganismen eine reversible oder irreversible Inaktivierung der enzymatischen Wirkung von Katalase ergibt, wobei es durch geeignete Wahl der erfindungsgemässen chemischen Verbindungen möglich ist, entweder nur aktive endogene Katalase oder sowohl letztere wie auch aktive intrazelluläre Katalase insbesondere von lebenden Mikroorganismen wie Bakterien und/oder Pilze, insbesondere Schimmelpilze und/oder Hefen, in ihrer enzymatischen Wirkung zu inaktivieren.

Erfindungsgemässe chemische Verbindungen, welche die aktive intrazelluläre Katalase von lebenden Mikroorganismen wie Bakterien und/oder Pilze, insbesondere Schimmelpilze und/oder Hefen, in ihrer enzymatischen Wirkung irreversibel inaktivieren, bewirken das Absterben dieser Mikroorganismen.

Nachstehend wird das Vorgehen zur Verwendung der erfindungsgemässen chemischen Verbindungen zur Messung einer Konzentration an lebenden und/oder aktiven Mikroorganismen wie Bakterien und/oder Pilze, insbesondere Schimmelpilze oder Hefen, beschrieben.

### Verfahren zur Messung einer Konzentration an lebenden und/oder aktiven Mikroorganismen in einer Probenflüssigkeit

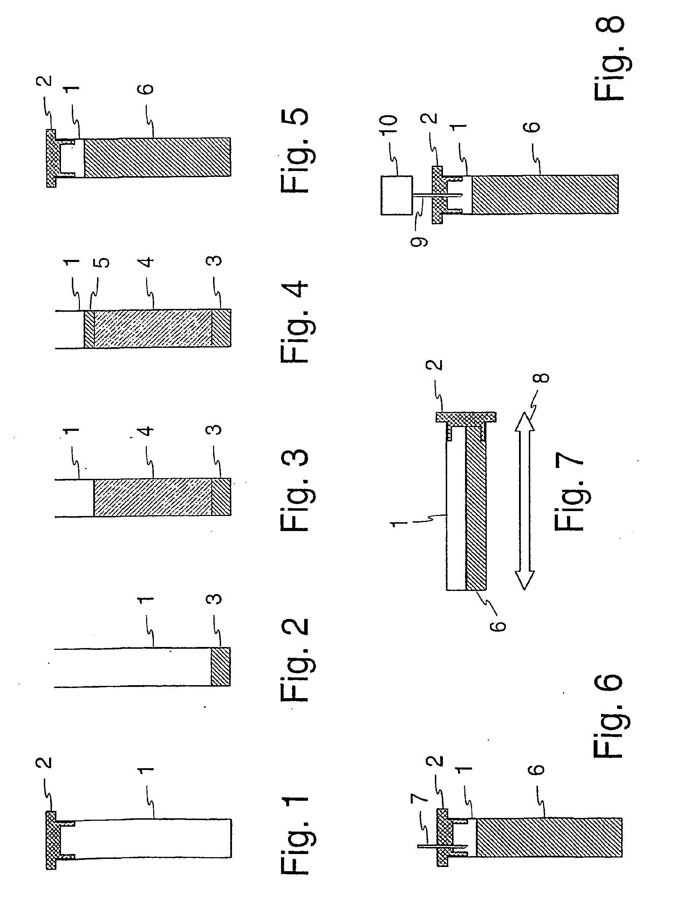

Das erfindungsgemässe Verfahren zur Messung einer Konzentration an lebenden und/oder aktiven Mikroorganismen in einer Probenflüssigkeit wird nachstehend anhand der Figuren 1 bis 8 beschrieben. Es beruht auf der Bestimmung der Entwicklung von Sauerstoff aus Wasserstoffperoxid, das der gegebenenfalls verdünnten und/oder gepufferten Probenflüssigkeit zugegeben und durch in der Probenflüssigkeit enthaltener Katalase schnell zu Sauerstoff und Wasser abgebaut wird.

Figur 1 zeigt einen bereitgestellten, im wesentlichen zylindrischen Behälter in einer Ausbildung als Reagenzglas 1, welches beispielsweise ein 15-ml-Röhrchen Vacutainer^{®} 16 x 125 mm von Becton Dickinson ist, und das mit einem darauf aufsetzbaren Stopfen 2 aus chemikalienbeständigem elastischem Kunststoff beispielsweise aus Viton^{®} von Du Pont de Nemours gasdicht verschliessbar ist. Zur Veranschaulichung ist in Figur 1 der Stopfen 2 auf das Reagenzglas 1 aufgesetzt dargestellt.

Figur 2 veranschaulicht, dass 1 ml der zu untersuchenden Probenflüssigkeit 3 in das Reagenzglas 1 eingesetzt wird. Nicht dargestellt ist dabei, dass die Probenflüssigkeit 3 gegebenenfalls aus einer ursprünglichen Probe durch Verdünnung und/oder Pufferung (vorzugsweise auf einen pH-Wert zwischen 6 und 7) gewonnen wurde.

Figur 3 veranschaulicht, dass der Probenflüssigkeit 3 nun 7 ml einer Reagenzflüssigkeit 4 zugemischt werden, die eine etwa 0,05 M wässrige Lösung des erfindungsgemässen Wirkstoffes d.h. einer erfindungsgemässen chemischen Verbindung oder eines Gemisches von erfindungsgemässen chemischen Verbindungen ist. Dabei wird, im Hinblick auf die angestrebte Messung einer Konzentration an lebenden und/oder aktiven Mikroorganismen, der erfindungsgemässe Wirkstoff so gewählt, dass er die enzymatische Wirkung von in der Probenflüssigkeit allenfalls vorhandener endogener Katalase inaktiviert, jedoch die enzymatische Wirkung von intrazellulärer Katalase der zur messenden Mikroorganismen nicht merklich inaktiviert.

Figur 4 veranschaulicht, dass den in Figur 3 veranschaulichten Flüssigkeitsmengen 3 und 4 nun, vorzugsweise durch dosiertes Eintropfen, etwa 0,2 ml (etwa 0,23 g) einer 30 Gew.-%igen wässrigen Wasserstoffperoxidlösung 5 zugemischt werden, worauf sich nach erfolgter Vermischung eine Konzentration von etwa 1 Vol.-% Wasserstoffperoxid im resultierenden (in Figur 5 veranschaulichten) Gemisch 6 einstellt. Gegebenenfalls ist es hilfreich, vor der Zugabe der Wasserstoffperoxidlösung einen oder einige wenige Tropfen Entschäumer zuzugeben.

Figur 5 veranschaulicht, dass sofort nach der in Figur 4 veranschaulichten Zugabe der Wasserstoffperoxidlösung 5 der Stopfen 2 auf das Reagenzglas 1 aufgesetzt wird, um das Gemisch 6 gasdicht darin zu verschliessen.

Figur 6 veranschaulicht, dass sofort nach dem in Figur 5 veranschaulichten gasdichten Verschliessen des Reagenzglases 1 mit dem Stopfen 2 letzterer mit einer Hohlnadel 7, welche beispielsweise eine Injektionsnadel Ø 1 mm ist, durchstochen wird, worauf sich der im Reagenzglas 1 herrschende Gasdruck dem atmosphärischen Druck angleicht. Nach wenigen, vorzugsweise 1 bis 2 Sekunden wird die Hohlnadel 7 aus dem Stopfen 2 zurückgezogen, worauf letzterer dank seiner Elastizität und weil er im Bereich des Reagenzglases 1 radial zusammengedrückt wird, einen von der zurückgezogenen Hohlnadel 7 hinterlassenen (nicht dargestellten) Durchstechkanal gasdicht verschliesst.

Figur 7 veranschaulicht, dass nun das Gemisch 6 im verschlossenen Reagenzglas 1 während einer vorbestimmten Reaktionszeit, die ab dem Zurückziehen der Hohlnadel 7 aus dem Stopfen 2 gezählt wird, vorzugsweise während 15 Minuten, gasdicht eingeschlossen bleibt und dabei im horizontal gekippten Reagenzglas 1 geschüttelt wird, vorzugsweise durch ein 10maliges kräftiges Hinundherschütteln, das durch den Doppelpfeil 8 symbolisiert wird. Während dieser Reaktionszeit findet die Entwicklung von Sauerstoff aus dem im Gemisch 6 enthaltenen Wasserstoffperoxid statt.

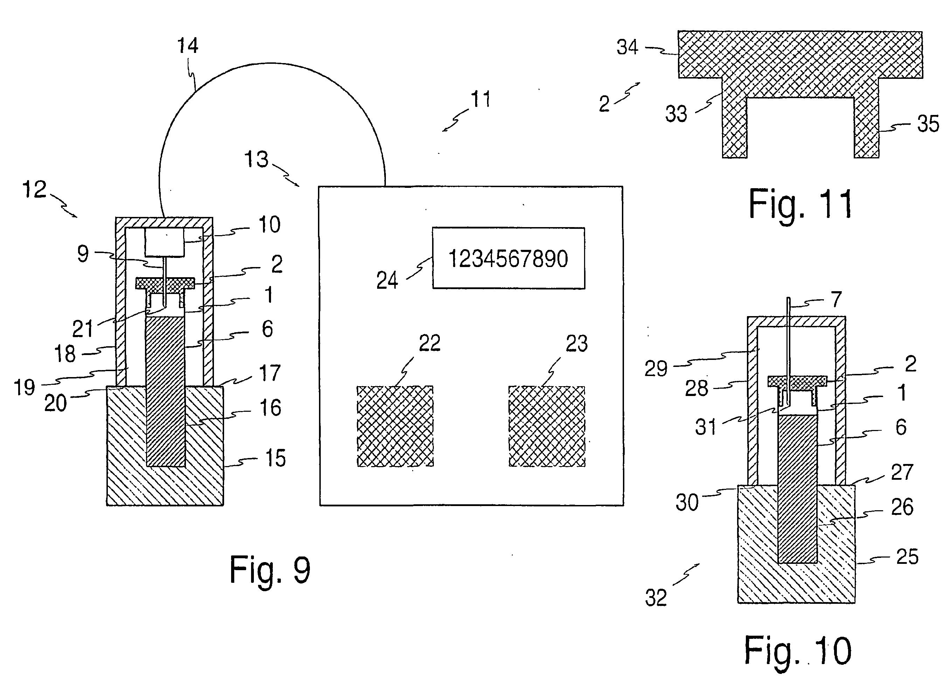

Figur 8 veranschaulicht, dass am Ende der vorbestimmten Reaktionszeit der im Reagenzglas 1 herrschende Druck gemessen wird, indem der Stopfen 2 von einer Hohlnadel 9, welche beispielsweise ebenfalls eine Injektionsnadel Ø 1 mm ist, durchstochen wird, wobei diese Hohlnadel 9 mit einem schematisch dargestellten Drucksensor 10 verbunden ist. Dieser Drucksensor 10 ist seinerseits Teil eines Druckmessgeräts 11, das weiter unten im Zusammenhang mit Figur 9 näher beschrieben wird.

Aus einem Vergleich der Figuren 6 und 8 ist erkennbar, dass der Stopfen 2 von der Hohlnadel 7 (zum Druckausgleich) und von der Hohlnadel 9 (zur Druckmessung) an verschiedenen Stellen durchgestochen wird, um ein Entweichen von Gas aus dem Reagenzglas 1 bei einem Einstechen der Hohlnadel 9 in den im Stopfen 2 von der zurückgezogenen Hohlnadel 7 hinterlassenen (nicht dargestellten) Durchstechkanal (und sich daraus ergebende Messfehler) zu vermeiden.

Bei der Ausführung des erfindungsgemässen Verfahrens ist normalerweise bekannt, ob und in welchem Verhältnis das untersuchte Gut bzw. die Probe davon verdünnt wurde, um die Probenflüssigkeit 3 zu ergeben. Auch ist normalerweise aus Erfahrung bekannt, welche Arten von Mikroorganismen im untersuchten Gut vorherrschend auftreten, so dass auch der Wirkungsgrad bzw. die Aktivität der im untersuchten Gut vorkommenden Katalase normalerweise bekannt ist. Mit Hilfe dieser Informationen und nachdem ein Blindwert sowie mit bekannten Konzentrationen gleichartiger Mikroorganismen eine Kennlinie des Druckmessgeräts 11 ermittelt worden sind, lässt sich aus dem gemessenen Druck die Konzentration an lebenden und/oder aktiven Mikroorganismen in der Probe errechnen. Es ist ein leichtes und braucht hier nicht näher beschrieben zu werden, im Druckmessgerät 11 einen Mikroprozessor 22 und Datenspeicher 23 vorzusehen (vgl. dazu die Beschreibung weiter unten im Zusammenhang mit Figur 9), um darin die erwähnten Daten zu speichern und so zu verarbeiten, dass die Konzentration an lebenden und/oder aktiven Mikroorganismen in der Probe automatisch errechnet und vorzugsweise direkt als Zahlenwert angezeigt wird.

### Vorrichtung zur Messung einer Konzentration an lebenden und/oder aktiven Mikroorganismen in einer Probenflüssigkeit

Figur 9 veranschaulicht ein gesamthaft mit 11 bezeichnetes Druckmessgerät einer Vorrichtung zur Ausführung des erfindungsgemässen Verfahrens. Das Druckmessgerät 11 umfasst einen schematisch dargestellten und gesamthaft mit 12 bezeichneten Drucksondenteil und einen schematisch dargestellten und gesamthaft mit 13 bezeichneten Druckauswerteteil, die über elektrische Leitungen eines abgeschirmten mehradrigen elektrischen Kabels 14 miteinander verbunden sind.

Der Drucksondenteil 12 umfasst einen Sockel 15 in Form einer zylindrischen blinden Hülse, deren Bohrung 16 beim Gebrauch vertikalachsig angeordnet ist. In diese Bohrung 16 passt das Reagenzglas 1 mit geringem Spiel, wobei es etwa bis zur Hälfte seiner Länge axial darin einführt werden kann. Ein ringförmiger Rand des Sockels 15 am Ende der Bohrung 16 bildet eine Anschlagfläche 17, die dem ganz in die Bohrung 16 eingesteckten Reagenzglas 1 zugeordnet und zu diesem in vorbestimmter Lage positioniert ist, unabhängig davon, ob und wie weit hinein ein Stopfen 2 in das Reagenzglas 1 eingeschoben ist, um letzteres zu verschliessen. Femer umfasst der Drucksondenteil 12 die bereits genannte Hohlnadel 9 und den bereits genannten Drucksensor 10 sowie eine zylindrische blinde Positionierhülse 18, deren Bohrung 19 beim Gebrauch vertikalachsig angeordnet ist. Koaxial zur Bohrung 19 sind die Hohlnadel 9 fest am Drucksensor 10 und dieser fest an der Positionierhülse 18 angeordnet. Ein ringförmiger Rand der Positionierhülse 18 am Ende der Bohrung 19 bildet eine Anschlagfläche 20 in fester Lage und Zuordnung zur Positionierhülse 18 und folglich über den Drucksensor 10 und die Hohlnadel 9 zu deren Spitze 21. Die axiale Länge der Positionierhülse 18 ist so bemessen, dass beim Gebrauch des Drucksondenteils 12 der Stopfen 2 soweit von der Hohlnadel 9 durchstochen wird, letztere jedoch nur soweit in das Innere des Reagenzglases 1 eingeführt werden kann, dass ihre Spitze 21 oberhalb des im Reagenzglas 1 enthaltenen Gemisches 6 in einem dort befindlichen Gasraum bleibt. Schliesslich sind, wie aus Figur 9 ersichtlich, der Drucksondenteil 12, das Reagenzglas 1 und deren zugeordnete Anschlagflächen 17 und 20 so bemessen und zueinander positioniert, dass beim Durchstechen des Stopfens 2 mit der Hohlnadel 9 nur diese mit dem Stopfen 2 in Kontakt kommt, so dass letzterer nur geringfügig und vorwiegend nur radial belastet und folglich nicht in das Innere des Reagenzglases 1 eingedrückt wird, was eine Volumenänderung und sich daraus ergebende Messfehler verursachen würde, die somit vermieden werden.

Der Druckauswerteteil 13 umfasst einen Mikroprozessor 22 und Datenspeicher 23, die vom Drucksensor 10 im Drucksondenteil 12, über das Kabel 14, Daten erhalten. Ausserdem sind zumindest ein Teil der Datenspeicher 23 (beispielsweise als EPROM, Flash-Memory oder sonstige auswechselbare und einsetzbare Speicher) und damit auch der Mikroprozessor 22 programmierbar. Somit können im Druckauswerteteil 13 Daten zum Blindwert des Druckmessgeräts 11 wie auch Daten zu einer Kennlinie des Druckmessgeräts 11 gespeichert werden. Die Bestimmung dieser Kennlinie erfolgt mit bekannten Konzentrationen an lebenden und/oder aktiven Mikroorganismen der gleichen Art wie diejenigen, die sich erwartungsgemäss in der Probenflüssigkeit 3 befinden, und in den Daten zur Kennlinie des Druckmessgeräts 11 können auch Daten zur Probenverdünnung subsumiert werden. Der so programmierte Mikroprozessor 22 errechnet einen Wert der Konzentration an lebenden und/oder aktiven Mikroorganismen in der Probe aus den Daten zum Druckwert, die vom Drucksondenteil 12 über das Kabel 14 geliefert werden, sowie aus den Daten, die der Mikroprozessor 22 aus den Datenspeichern 23 erhält. Dank dieser automatisierten und progammierten Errechnung ist es möglich, einen errechneten Wert zu erhalten, der direkt als Zahlenwert die Konzentration an lebenden und/oder aktiven Mikroorganismen in der Probe darstellt und im Druckauswerteteil 13 auf einer Anzeige 24 erscheint.

Figur 10 veranschaulicht ein schematisch dargestelltes und gesamthaft mit 32 bezeichnetes Entlüftungswerkzeug einer Vorrichtung zur Ausführung des erfindungsgemässen Verfahrens. Einige Teile in diesem Entlüftungswerkzeug 32 sind entsprechenden Teilen im Drucksondenteil 12 gleich, es kann sich sogar um dieselben Teile in anderer Funktion handeln: solche äquivalente Teile werden hier nur mehr erwähnt, es wird auf die weiter oben gegebene Beschreibung ihrer Äquivalente im Zusammenhang mit dem Drucksondenteil 12 verwiesen.

Das Entlüftungswerkzeug 32 umfasst einen Sockel 25, der dem Sockel 15 gleich ist. In eine Bohrung 26, die der Bohrung 16 gleich ist und beim Gebrauch ebenfalls vertikalachsig angeordnet ist, passt das Reagenzglas 1 auf gleiche Weise. Ein ringförmiger Rand des Sockels 25 am Ende der Bohrung 26 bildet eine Anschlagfläche 27, die der Anschlagfläche 17 gleich ist. Ferner umfasst das Entlüftungswerkzeug 32 die bereits genannte Hohlnadel 7 sowie eine zylindrische blinde Positionierhülse 28, deren Bohrung 29 beim Gebrauch vertikalachsig angeordnet ist. Parallel zur Achse der Bohrung 29 aber nicht koaxial dazu d.h. exzentrisch ist die Hohlnadel 7 fest an der Positionierhülse 28 angeordnet. Ein ringförmiger Rand der Positionierhülse 28 am Ende der Bohrung 29 bildet eine Anschlagfläche 30 in fester Lage und Zuordnung zur Positionierhülse 28 und folglich über die Hohlnadel 7 zu deren Spitze 31. Die axiale Länge der Positionierhülse 28 ist so bemessen, dass beim Gebrauch des Entlüftungswerkzeugs 32 der Stopfen 2 nur soweit von der Hohlnadel 7 durchstochen wird und letztere nur soweit in das Innere des Reagenzglases 1 eingeführt werden kann, dass ihre Spitze 31 oberhalb des im Reagenzglas 1 enthaltenen Gemisches 6 in einem dort befindlichen Gasraum bleibt. Schliesslich sind, wie aus Figur 10 ersichtlich, das Entlüftungswerkzeug 32, das Reagenzglas 1 und deren zugeordnete Anschlagflächen 27 und 30 so bemessen und zueinander positioniert, dass beim Durchstechen des Stopfens 2 mit der Hohlnadel 7 nur diese mit dem Stopfen 2 in Kontakt kommt, so dass letzterer nur geringfügig und vorwiegend nur radial belastet und folglich nicht in das Innere des Reagenzglases 1 eingedrückt wird, was eine Volumenänderung und sich daraus ergebende Messfehler verursachen würde, die somit vermieden werden.

Figur 11 veranschaulicht Einzelheiten des schematisch im axialen Längsschnitt dargestellten Stopfens 2. Um zu gewährleisten, dass der Stopfen 2 einerseits das Reagenzglas 1 gasdicht zu verschliessen vermag, andererseits von den Hohlnadeln 7 und 9 durchstochen werden kann, ist dieser Stopfen 2 aus einem chemikalienbeständigem elastischem Kunststoff hergestellt und im wesentlichen pilzförmig ausgebildet mit einem in das Reagenzglas 1 einführbaren Zylinder 33, an dem oben ein Kopf 34 und unten eine hülsenförmiger Dichtungslippe 35 angeformt sind. Bei mit dem Stopfen 2 verschlossenen Reagenzglas 1 liegen, radial komprimiert, der Zylinder 34 und die Dichtungslippe 35 voll innerhalb des Reagenzglases 1, während der etwas breitere Kopf 33 ausserhalb des Reagenzglases 1 bleibt und dessen Mündung überdeckt. Wenn beispielsweise das Reagenzglas 1 ein 15-ml-Röhrchen Vacutainer^{®} 16 x 125 mm von Becton Dickinson ist und der Stopfen 2 aus Viton^{®} von Du Pont de Nemours besteht, beträgt die gesamte von den Hohlnadeln 7 und 9 durchzustechende Höhe des Stopfens 2 etwa 5 mm, und der Zylinder 34 wird radial um etwa 7% komprimiert.

Im vorstehenden wurde die Erfindung anhand einzelner Beispiele beschrieben, die sich auf die Herstellung der erfindungsgemässen Substanzen und die Herbeiführung und/oder Verwendung einer Modifikation der Kinetik der enzymatischen Wirkung von Katalase bezogen. Es bieten sich viele Einsatzmöglichkeiten für die erfindungsgemässen Substanzen, für deren Verwendung zur Modifikation der Kinetik der enzymatischen Wirkung von Katalase und gegebenenfalls zur Abtötung von Mikroorganismen, für das erfindungsgemässe Verfahren zur Messung einer Konzentration an lebenden und/oder aktiven Mikroorganismen und für die entsprechende erfindungsgemässe Vorrichtung. Es können, in einer Auflistung von Beispielen ohne jegliche Einschränkung darauf, als Einsatzmöglichkeiten zur Messung, zur Kontrolle und/oder zur Verfahrenssteuerung, sowie zur Abtötung von Mikroorganismen, genannt werden: bei Lebensmitteln auf Stufe Konsum, Handel und Industrie (gegen mikrobiellen Verderb); bei der spanabhebenden Werkstoffverarbeitung (bezüglich wassermischbare Kühlschmierstoffe usw.); bei der Humanmedizin und der Tiermedizin insbesondere bei der Versorgung und Entsorgung in Spitälern und medizinischen Labors (Blut, Urin, Anlagen und Instrumente, Verbandzeug usw.); bei der Frischwasserversorgung, der Wasservorratshaltung und der Abwasserklärung; bei Lüftungs- und Klimaanlagen (Frischluftreinigung, Luftzirkulation, Abluftentsorgung); und bei der Forschung und Entwicklung auf den vorgenannten oder anderen Gebieten.

Zusammenfassend kann festgehalten werden, dass mit dem dargestellten und beschriebenen erfindungsgemässen Katalase-Verfahren
a) auch sehr niedrige Keimzahlen aus einer Probe durch Filtration mittels geeigneter Membranfilter nachgewiesen werden können;
b) eine Differenzierung zwischen Bakterien einerseits, Pilze und Hefen andererseits, durch Filtration mittels geeigneter Membranfilter möglich ist; und
c) eine höhere Empfindlichkeit der Messwerte erreicht wird, weil im Vergleich zum Bisherigen empfindlichere Druckaufnehmer-Sonden verwendet werden können;
und dass die erfindungsgemässe Verbindung sowohl aktive endogene Katalase als auch aktive bakterielle Katale inaktiviert und als aktive Desinfektionskomponente allein oder für die weitere Einarbeitung in Desinfektionsmitteln zu Abtötung von Mikroorganismen verwendbar ist.

### Liste der Bezugszeichen

- 1: Reagenzglas
- 2: Stopfen
- 3: Probenflüssigkeit
- 4: Reagenzflüssigkeit
- 5: Wasserstoffperoxidlösung
- 6: Gemisch
- 7: Hohlnadel zum Druckausgleich
- 8: Hinundherschütteln
- 9: Hohlnadel zur Druckmessung
- 10: Drucksensor
- 11: Druckmessgerät
- 12: Drucksondenteil
- 13: Druckauswerteteil
- 14: elektrisches Kabel
- 15: Sockel des Drucksondenteils 12
- 16: Bohrung im Sockel 15
- 17: Anschlagfläche am Sockel 15
- 18: Positionierhülse
- 19: Bohrung der Positionierhülse 18
- 20: Anschlagfläche an der Positionierhülse 18
- 21: Spitze der Hohlnadel 9
- 22: Mikroprozessor
- 23: Datenspeicher
- 24: Anzeige
- 25: Sockel des Entlüftungswerkzeugs 32
- 26: Bohrung im Sockel 25
- 27: Anschlagfläche am Sockel 25
- 28: Positionierhülse
- 29: Bohrung der Positionierhülse 28
- 30: Anschlagfläche an der Positionierhülse 18
- 31: Spitze der Hohlnadel 7
- 32: Entlüftungswerkzeug
- 33: Zylinder des Stopfens 2
- 34: Kopf des Stopfens 2
- 35: Dichtungslippe des Stopfens 2

## Patentansprüche

1. Sulfonylester der allgemeinen Formel
R₁-COO-SO₂-Z-R₂
worin
Z für Phenylen steht oder abwesend ist,
R1 über ein primäres oder sekundäres Kohlenstoffatom mit dem Kohlenstoffatom des Säureradikals -COO- verbunden ist und für geradkettiges oder verzweigtes Alkyl mit 2 bis 21000 Kohlenstoffatomen, geradkettiges oder verzweigtes Alkenyl mit 2 bis 21000 Kohlenstoffatomen, oder geradkettiges oder verzweigtes Alkinyl mit 2 bis 21000 Kohlenstoffatomen steht, und
R₂ über ein sekundäres oder tertiäres Kohlenstoffatom mit dem Radikal Phenylen in Position ortho, meta oder para zum Radikal SO₂, wenn Z vorhanden ist, oder mit dem Radikal SO₂, wenn Z abwesend ist, verbunden ist und für geradkettiges oder verzweigtes Alkyl mit 3 bis 100 Kohlenstoffatomen, geradkettiges oder verzweigtes Alkenyl mit 3 bis 100 Kohlenstoffatomen, geradkettiges oder verzweigtes Alkinyl mit 3 bis 100 Kohlenstoffatomen, geradkettiges oder verzweigtes Alkoxy mit 3 bis 100 Kohlenstoffatomen, geradkettiges oder verzweigtes Hydroxyalkyl mit 3 bis 100 Kohlenstoffatomen,
wobei das Radikal Hydroxy über ein primäres oder sekundäres Kohlenstoffatom mit dem Radikal Alkyl verbunden ist, oder
Polyoxaalkyl mit 3 bis 100 Kohlenstoffatomen steht.

2. Verbindung nach Anspruch 1, welche eine aus der von 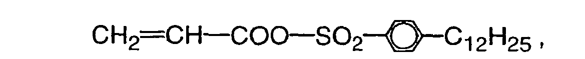 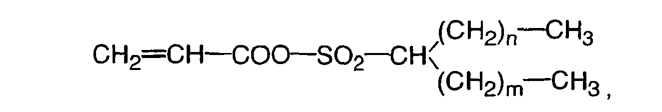 worin n und m natürliche Zahlen sind und 3 < (n+m) < 60 ,
CH₂=CH-COO-SO₂-CH₂-CH=CH-C₁₃H₂₇,
CH₂=CH-COO-SO₂-O-C₁₂H₂₅, 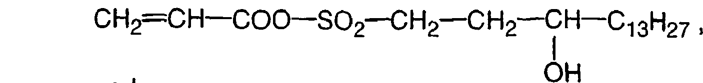
und
CH₂=CH-COO-SO₂-O-CH₂-CH₂-O-CH₂-CH₂-O-C₁₂H₂₅
gebildeten Gruppe ausgewählt ist.

3. Verwendung einer Verbindung nach einem der Ansprüche 1 oder 2 zur Modifikation der Kinetik der enzymatischen Wirkung von Katalase.

4. Verwendung nach Anspruch 3, **dadurch gekennzeichnet, dass** die Modifikation der Kinetik eine Inaktivierung ist.

5. Verwendung nach Anspruch 4, **dadurch gekennzeichnet, dass** die Inaktivierung irreversibel ist.

6. Verwendung nach einem der Ansprüche 4 und 5, **dadurch gekennzeichnet, dass** sich die Inaktivierung auf die enzymatische Wirkung von aktiver endogener Katalase bezieht.

7. Verwendung nach Anspruch 6, **dadurch gekennzeichnet, dass** sich die Inaktivierung auf die enzymatische Wirkung von aktiver endogener Katalase bezieht, ohne dass eine merkliche Inaktivierung der enzymatischen Wirkung von aktiver intrazellulärer Katalase erfolgt.

8. Verwendung nach Anspruch 7, **dadurch gekennzeichnet, dass** die aktive intrazelluläre Katalase diejenige von lebenden Mikroorganismen ist.

9. Verwendung nach Anspruch 8, **dadurch gekennzeichnet, dass** die lebenden Mikroorganismen Bakterien und/oder Pilze, insbesondere Schimmelpilze und/oder Hefen sind.

10. Verwendung nach einem der Ansprüche 3 bis 9 zur Abtötung von Mikroorganismen.

11. Verfahren zur Messung einer Konzentration an lebenden und/oder aktiven Mikroorganismen wie Bakterien und/oder Pilze, insbesondere Schimmelpilze und/oder Hefen, in einer flüssigen Probe anhand der Entwicklung von Sauerstoff aus Wasserstoffperoxid, das der gegebenenfalls verdünnten und/oder gepufferten Probe zugegeben wird, wobei die Sauerstoffentwicklung durch in der Probe enthaltener Katalase herbeigeführt wird, **dadurch gekennzeichnet, dass**
a) der in einem gasdicht verschliessbaren Behälter enthaltenen Probe eine Verbindung nach Anspruch 1 zugemischt wird, welche die enzymatische Wirkung von endogener Katalase inaktiviert, ohne die enzymatische Wirkung von intrazellulärer Katalase von Mikroorganismen merklich zu inaktivieren;
b) dem beim Schritt (a) hergestellten Gemisch Wasserstoffperoxid zugemischt wird;
c) sofort nach dem Schritt (b) der Behälter gasdicht verschlossen wird;
d) sofort nach dem Schritt (c) ein im Behälter herrschender Gasdruck dem atmosphärischen Druck angeglichen wird;
e) sofort nach dem Schritt (d) der Behälter wieder gasdicht verschlossen wird;
f) der Behälter ab dessen Abdichtung beim Schritt (e) während einer vorbestimmten Reaktionszeit gasdicht verschlossen bleibt; und
g) am Ende der vorbestimmten Reaktionszeit ein im Behälter herrschender Druck gemessen wird.

12. Verfahren nach Anspruch 11, **dadurch gekennzeichnet, dass** beim Schritt (d) der Druckausgleich und beim Schritt (g) die Druckmessung mit Hilfe je einer Hohlnadel erfolgt, mit der ein den Behälter gasdicht verschliessenden Stopfen an bei den Schritten (d) und (g) verschiedenen Stellen durchgestochen wird.

13. Verfahren nach Anspruch 11, **dadurch gekennzeichnet, dass** aus dem gemessenen Druck sowie aus den Mikroorganismen entsprechenden Daten und aus Daten zur Probenverdünnung die Konzentration an lebenden und/oder aktiven Mikroorganismen in der Probe errechnet wird.

14. Verfahren nach Anspruch 13, **dadurch gekennzeichnet, dass** die Konzentration an lebenden und/oder aktiven Mikroorganismen angezeigt wird.

15. Vorrichtung zur Ausführung des Verfahrens nach einem der Ansprüche 11 bis 14, **dadurch gekennzeichnet, dass** der Behälter (1) im wesentlichen zylindrisch ausgebildet und mit einem Stopfen (2) gasdicht verschlossen ist, unabhängig vom Stopfen eine dem Behälter feststehend zugeordnete Anschlagfläche (17) vorgesehen ist, und ein Drucksondenteil (12) vorgesehen ist, welcher eine mit einem Drucksensor (10) verbundene Hohlnadel (9) und eine mit einer Anschlagfläche (20) versehene Positionierhülse (18) aufweist, derart, dass bei der Benutzung des Drucksondenteils dessen Positionierhülse auf den Behälter aufsetzbar ist und die Hohlnadel beim Durchstechen des Stopfens durch diesen hindurch vorschiebbar ist, bis die beiden genannten Anschlagflächen (18, 20) aneinander anliegen, worauf eine Spitze (21) der Hohlnadel (9) in eine vorbestimmte Lage zum Behälter zu liegen kommt.

16. Vorrichtung nach Anspruch 15, **dadurch gekennzeichnet, dass** ein als blinde zylindrische Hülse mit ringförmigem Rand ihrer Bohrung (16, 26) ausgebildeter Sockel (15, 25) vorgesehen ist, in den der Behälter (1) einsetzbar ist, derart, dass der genannte Rand die dem Behälter zugeordnete Anschlagfläche (17, 27) bildet.

17. Vorrichtung nach einem der Ansprüche 15 und 16, **dadurch gekennzeichnet, dass** der Drucksondenteil (12), der Behälter (1) und deren zugeordnete Anschlagflächen (17, 27) so bemessen und zueinander positioniert sind, dass nur die Hohlnadel (9) mit dem Stopfen (2) in Kontakt kommt.

18. Vorrichtung nach einem der Ansprüche 15 bis 17, **gekennzeichnet durch** ein Druckauswerteteil (13) mit Einrichtungen (22, 23, 24) zur Errechnung eines Wertes der Konzentration an lebenden und/oder aktiven Mikroorganismen in der Probe aus einem vom Drucksensor erfassten Druckwert und aus gespeicherten, der Art der gemessenen Mikroorganismen sowie der Probenverdünnung entsprechenden Daten, und zur Ausgabe des errechneten Wertes.

19. Vorrichtung nach einem der Ansprüche 15 bis 17, **gekennzeichnet durch** einen Stopfen (2) aus chemikalienbeständigem elastischem Kunststoff, mit welchem der Behälter (1) gasdicht verschliessbar ist und welcher von der Hohlnadel (7, 9) durchstechbar ist.

## Claims

1. Sulfonyl esters of the general formula
R₁-COO-SO₂-Z-R₂
in which
Z is phenylene or is absent,
R₁ is connected via a primary or secondary carbon atom to the carbon atom of the acid radical -COO- and is straight-chain or branched alkyl having 2 to 21000 carbon atoms, straight-chain or branched alkenyl having 2 to 21000 carbon atoms, or straight-chain or branched alkynyl having 2 to 21000 carbon atoms, and
R₂ is connected via a secondary or tertiary carbon atom to the phenylene radical in the position ortho, meta or para to the SO₂ radical when Z is present, or to the SO₂ radical when Z is absent, and is straight-chain or branched alkyl having 3 to 100 carbon atoms, straight-chain or branched alkenyl having 3 to 100 carbon atoms, straight-chain or branched alkynyl having 3 to 100 carbon atoms, straight-chain or branched alkoxy having 3 to 100 carbon atoms, straight-chain or branched hydroxyalkyl having 3 to 100 carbon atoms,
where the hydroxyl radical is connected via a primary or secondary carbon atom to the alkyl radical, or
polyoxaalkyl having 3 to 100 carbon atoms.

2. A compound as claimed in claim 1, which is one selected from the group formed by 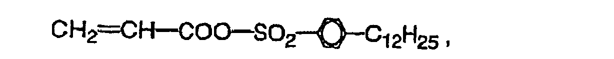 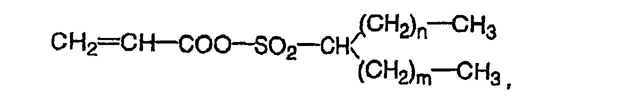 in which n and m are natural numbers and 3<(n+m)< 60,
CH₂=CH-COO-SO₂-CH₂-CH=CH-C₁₃H₂₇,
CH₂=CH-COO-SO₂-O-C₁₂H₂₅, 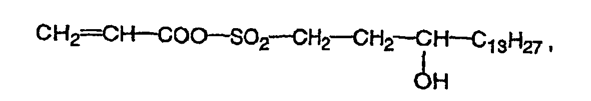
and
CH₂=CH-COO-SO₂-O-CH₂-CH₂-O-CH₂-CH₂-O-C₁₂H₂₅.

3. The use of a compound as claimed in either of claims 1 or 2 for modifying the kinetics of the enzymatic action of catalase.

4. The use as claimed in claim 3, **characterized in that** the modification of the kinetics is an inactivation.

5. The use as claimed in claim 4, **characterized in that** the inactivation is irreversible.

6. The use as claimed in either of claims 4 and 5, **characterized in that** the inactivation relates to the enzymatic action of active endogenous catalase.

7. The use as claimed in claim 6, **characterized in that** the inactivation relates to the enzymatic action of active endogenous catalase without a noticeable inactivation of the enzymatic action of active intracellular catalase taking place.

8. The use as claimed in claim 7, **characterized in that** the active intracellular catalase is that of live microorganisms.

9. The use as claimed in claim 8, **characterized in that** the live microorganisms are bacteria and/or fungi, in particular molds and/or yeasts.

10. The use as claimed in any of claims 3 to 9 for killing microorganisms.

11. A method for measuring a concentration of live and/or active microorganisms such as bacteria and/or fungi, in particular molds and/or yeasts, in a liquid sample by means of the evolution of oxygen from hydrogen peroxide which is added to the sample which has been diluted and/or buffered
where appropriate, the oxygen evolution being brought about by catalase present in the sample, **characterized in that**
a) a compound as claimed in claim 1 which inactivates the enzymatic action of endogenous catalase without noticeably inactivating the enzymatic action of intracellular catalase of microorganisms is admixed to the sample present in a container which can be closed gas-tight;
b) hydrogen peroxide is admixed to the mixture prepared in step (a);
c) the container is closed gas-tight immediately after step (b);
d) a gas pressure prevailing in the container is made equal to atmospheric pressure immediately after step (c);
e) the container is closed gas-tight again immediately after step (d);
f) the container remains closed gas-tight for a predetermined reaction time after its sealing in step (e); and
g) a pressure prevailing in the container at the end of the predetermined reaction time is measured.

12. The method as claimed in claim 11, **characterized in that** the pressure equalization in step (d) and the pressure measurement in step (g) each takes place with the aid of a hollow needle with which a stopper closing the container gas-tight is pierced at different places in steps (d) and (g).

13. The method as claimed in claim 11, **characterized in that** the concentration of live and/or active microorganisms in the sample is calculated from the measured pressure and from data corresponding to the microorganisms and from data on the sample dilution.

14. The method as claimed in claim 13, **characterized in that** the concentration of live and/or active microorganisms is displayed.

15. An apparatus for carrying out the method as claimed in any of claims 11 to 14, **characterized in that** the container (1) is designed to be substantially cylindrical and is closed gas-tight with a stopper (2), independently of the stopper a stop face (17) is provided in fixed correlation with the container, and a pressure probe part (12) which has a hollow needle (9) connected to a pressure sensor (10) and has a positioning socket (18) provided with a stop face (20) is provided in such a way that, on use of the pressure probe part, its positioning socket can be placed on the container, and the hollow needle can, on piercing the stopper, be advanced through the latter until the two stop faces (18, 20) mentioned are in contact with one another, after which a tip (21) of the hollow needle (9) is located in a predetermined position relative to the container.

16. The apparatus as claimed in claim 15, **characterized in that** a base (15, 25) which is designed as blind cylindrical socket with annular rim of its bore (16, 26) is provided, into which the container (1) can be inserted in such a way that said rim forms the stop face (17, 27) coordinated with the container.

17. The apparatus as claimed in either of claims 15 and 16, **characterized in that** the pressure probe part (12), the container (1) and the stop faces (17, 27) coordinated therewith are dimensioned and are positioned relative to one another such that only the hollow needle (9) comes into contact with the stopper (2).

18. The apparatus as claimed in any of claims 15 to 17, **characterized by** a pressure evaluation part (13) with devices (22, 23, 24) for calculating a value of the concentration of live and/or active microorganisms in the sample from a pressure detected by the pressure sensor and from stored data corresponding to the type of microorganisms measured and to the sample dilution, and for outputting the calculated value.

19. The apparatus as claimed in any of claims 15 to 17, **characterized by** a stopper (2) made of chemical-resistant elastic plastic with which the container (1) can be sealed gas-tight and which can be pierced by the hollow needle (7, 9).

## Revendications

1. Sulfonylester suivant la formule générale
R₁-COO-SO₂-Z-R₂
dans laquelle
Z représente le phénylène ou est absent,
R₁ est relié, par l'intermédiaire d'un atome de carbone primaire ou secondaire, à l'atome de carbone du radical acide -COO- et représente un alkyle linéaire ou ramifié comportant de 2 à 21000 atomes de carbone, un alcényle linéaire ou ramifié comportant de 2 à 21000 atomes de carbone ou un alcynyle linéaire ou ramifié comportant de 2 à 21000 atomes de carbone,
R₂ est relié, par l'intermédiaire d'un atome de carbone secondaire ou tertiaire, au radical phénylène en position ortho, méta ou para vis-à-vis du radical SO₂, lorsque Z est présent, ou au radical SO₂ lorsque Z est absent, et représente un alkyle linéaire ou ramifié comportant de 3 à 100 atomes de carbone, un alcényle linéaire ou ramifié comportant de 3 à 100 atomes de carbone, un alcynyle linéaire ou ramifié comportant de 3 à 100 atomes de carbone, un alcoxy linéaire ou ramifié comportant de 3 à 100 atomes de carbone,
un hydroxyalkyle linéaire ou ramifié comportant de 3 à 100 atomes de carbone, et dont le radical hydroxy est relié au radical alkyle par l'intermédiaire d'un atome de carbone primaire ou secondaire,
ou un polyoxyalkyle comportant de 3 à 100 atomes de carbone.

2. Composé selon la revendication 1, sélectionné au sein d'un groupe formé par 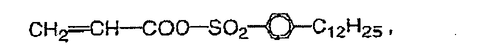 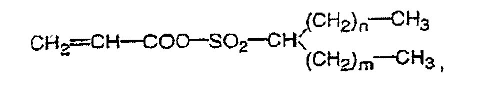 où n et m sont des nombres naturels et 3 < (n + m) < 60,
CH₂=CH-COO-SO₂-CH₂-CH=CH-C₁₃H₂₇.
CH₂=CH-COO-SO₂-O-C₁₂H₂₅. 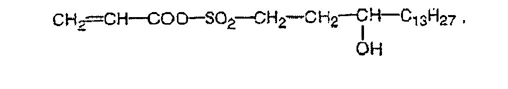
et
CH₂=CH-COO-SO₂-O-CH₂-CH₂-O-CH₂-CH₂-O-C₁₂H₂₅

3. Utilisation d'un composé selon l'une des revendications 1 ou 2 en vue de la modification de la cinétique de l'effet enzymatique de la catalase.

4. Utilisation selon la revendication 3,
**caractérisée en ce que**
la modification de la cinétique est une désactivation.

5. Utilisation selon la revendication 4,
**caractérisée en ce que**
la désactivation est irréversible.

6. Utilisation selon l'une des revendications 4 ou 5,
**caractérisée en ce que**
la désactivation concerne l'effet enzymatique de la catalase endogène active.

7. Utilisation selon la revendication 6,
**caractérisée en ce que**
la désactivation concerne l'effet enzymatique de la catalase endogène active, sans que s'effectue une désactivation notable de l'effet enzymatique de la catalase intracellulaire active.

8. Utilisation selon la revendication 7,
**caractérisée en ce que**
la catalase intracellulaire active est celle de micro-organismes vivants.

9. Utilisation selon la revendication 8,
**caractérisée en ce que**
les micro-organismes vivants sont des bactéries et/ou des champignons, et en particulier des moisissures et/ou des levures.

10. Utilisation selon l'une des revendications 3 à 9 pour l'extinction de micro-organismes.

11. Procédé pour la mesure d'une concentration de micro-organismes vivants et/ou actifs, tels que bactéries et/ou champignons, en particulier moisissures et/ou levures, dans un échantillon liquide à l'aide de la formation d'oxygène à partir de peroxyde d'hydrogène qui est ajouté à l'échantillon le cas échéant dilué et/ou tamponné, la formation d'oxygène étant causée par la catalase contenue dans l'échantillon,
**caractérisé en ce que**
a) à l'échantillon contenu dans un récipient pouvant être fermé de manière étanche au gaz, on ajoute un composé selon la revendication 1, lequel désactive l'effet enzymatique de la catalase endogène sans désactiver notablement l'effet enzymatique de la catalase intracellulaire de micro-organismes ;
b) au mélange produit à l'étape (a), on ajoute du peroxyde d'hydrogène;
c) aussitôt après l'étape (b), on ferme le récipient de manière étanche au gaz ;
d) aussitôt après l'étape (c), on ajuste une pression gazeuse régnant dans le récipient à la pression atmosphérique ;
e) aussitôt après l'étape (d), on referme le récipient de manière étanche au gaz ;
f) après son étanchéification à l'étape (e), le récipient reste fermé de manière étanche au gaz durant un temps de réaction prédéterminé ;
g) à la fin du temps de réaction prédéterminé, on mesure une pression régnant dans le récipient.

12. Procédé selon la revendication 11,
**caractérisé en ce que**
l'équilibrage de pression à l'étape (d) et la mesure de pression à l'étape (g) s'effectuent l'un et l'autre à l'aide d'une aiguille creuse par laquelle un bouchon, fermant le récipient de manière étanche au gaz, est percé en des endroits différents pour les étapes (d) et (g).

13. Procédé selon la revendication 11,
**caractérisé en ce qu'**
à partir de la pression mesurée, ainsi qu'à partir des données correspondant aux micro-organismes et de données concernant la dilution de l'échantillon, on calcule la concentration de micro-organismes vivants et/ou actifs dans l'échantillon.

14. Procédé selon la revendication 13,
**caractérisé en que**
la concentration de micro-organismes vivants et/ou actifs est indiquée.

15. Dispositif en vue de l'exécution du procédé selon l'une des revendications 11 à 14,
**caractérisé en ce que**
le récipient (1) est constitué pour l'essentiel de manière cylindrique et est fermé de manière étanche au gaz à l'aide d'un bouchon (2) ; indépendamment du bouchon, on prévoit une surface d'arrêt (17) associée fixement au récipient ; on prévoit aussi un élément de sonde manométrique (12) qui présente une aiguille creuse (9) reliée à un capteur de pression (10) et une douille de positionnement (18) pourvue d'une surface d'arrêt (20), de telle manière que, lors de l'utilisation de l'élément de sonde manométrique, sa douille de positionnement peut être posée sur le récipient et que, lors de la perforation du bouchon, l'aiguille creuse peut se déplacer à travers celui-ci jusqu'à ce que les deux surfaces d'arrêt précitées (17, 20) adhèrent l'une à l'autre, après quoi une pointe (21) de l'aiguille creuse (9) vient se situer dans une position prédéterminée par rapport au récipient.

16. Dispositif selon la revendication 15,
**caractérisé en ce qu'**
on prévoit un socle (15, 25), constitué sous forme de douille cylindrique aveugle comportant un bord annulaire de son alésage (16, 26) et dans lequel le récipient (1) peut être inséré, de telle manière que le bord précité forme la surface d'arrêt (17, 27) associée au récipient.

17. Dispositif selon l'une des revendications 15 et 16,
**caractérisé en ce que**
l'élément de sonde de pression (12), le récipient (1) et leurs surfaces d'arrêt associées (17, 27) sont dimensionnés et positionnés les uns vis-à-vis des autres de telle manière que seule l'aiguille creuse (9) entre en contact avec le bouchon (2).

18. Dispositif selon l'une des revendications 15 à 17,
**caractérisé par**
un appareil d'exploitation de pression (13) comportant des dispositifs (22, 23, 24) en vue du calcul d'une valeur de la concentration de micro-organismes vivants et/ ou actifs dans l'échantillon à partir d'une valeur de pression détectée par le capteur de pression et à partir de données enregistrées, correspondant à la nature des micro-organismes mesurés et à la dilution de l'échantillon, et en vue de la sortie de la valeur calculée.

19. Dispositif selon l'une des revendications 15 à 17,
**caractérisé par**
un bouchon (2) en matière plastique élastique, résistant aux agents chimiques, qui peut fermer le récipient (1) de manière étanche au gaz et être perforé par l'aiguille creuse (7, 9).
